(19) Europäisches Patentamt
European Patent Office
Office européen des brevets

(11) Numéro de publication : **0 004 968**
**B1**

(12) # FASCICULE DE BREVET EUROPÉEN

(45) Date de publication du fascicule du brevet : **21.10.81**

(51) Int. Cl.[3] : **C 07 D303/02**, C 11 B 9/00, A 61 K 7/46

(21) Numéro de dépôt : **79101172.9**

(22) Date de dépôt : **17.04.79**

(54) **Dérivés du norbornène(-ane), procédé pour la préparation de ces derivés et leur utilisation en tant qu'ingrédients parfumants.**

(30) Priorité : **17.04.78 CH 4073/78**

(43) Date de publication de la demande : **31.10.79 (Bulletin 79/22)**

(45) Mention de la délivrance du brevet : **21.10.81 Bulletin 81/42**

(84) Etats contractants désignés : **CH DE FR GB IT NL SE**

(56) Documents cités :
**DE - A - 2 163 770**
**DE - A - 2 617 816**
**FR - A - 1 416 209**

(73) Titulaire : **FIRMENICH SA**
**Case postale 239**
**CH-1211 Genève 8 (CH)**

(72) Inventeur : **Delay, Francois, Dr.**
**9, rue des Pervenches**
**CH-1227 Carouge/GE (CH)**

(74) Mandataire : **Schmied-Kowarzik, Volker, Dr. et al**
**Patentanwälte P. Wirth V. Schmied-Kowarzik G. Dannenberg P. Weinhold D. Gudel S. Schubert Siegfriedstrasse 8**
**D-8000 München 40 (DE)**

**EP 0 004 968 B1**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Imprimerie Jouve, 17, rue du Louvre, 75001 Paris, France

Dérivés du norbornène(-ane), procédé pour la préparation de ces dérivés et leur utilisation en tant qu'ingrédients parfumants.

L'invention se rapporte au domaine de la parfumerie et plus particulièrement à des composés de formule

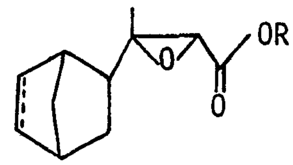

(Ia,b)

comportant une liaison simple ou double dans la position indiquée par les pointillés et dans laquelle le symbole R représente un radical alkyle inférieur comprenant 1 à 4 atomes de carbone.

La présente invention concerne, en outre l'utilisation des composés de formule (Ia, b) en tant qu'ingrédients parfumants pour la préparation de parfums et produits parfumés.

Elle concerne également une composition parfumante contenant, en tant qu'ingrédient actif, au moins un des composés de formule (Ia, b).

L'invention a enfin pour objet un procédé pour la préparation des composés de formule (Ia), comportant une liaison double dans la position indiquée par les pointillés, ledit procédé étant caractérisé en ce qu'on traite l'acétylnorbornène avec un chloracétate d'alkyle de formule

$$Cl-CH_2-CO_2R \qquad (II)$$

dans laquelle R représente un radical alkyle inférieur comprenant 1 à 4 atomes de carbone, en présence d'une base forte.

Les composés de formule (Ia) ainsi préparés peuvent être soumis à une hydrogénation catalytique pour fournir les dérivés saturés correspondants de formule (Ib).

La formule (Ia, b) sert donc à définir des esters de l'acide glycidique, en particulier et de façon préférentielle les esters méthylique et éthylique, ou 3-méthyl-3-(norbornène-5-yle)-glycidate de méthyle et 3-méthyl-3-(norbornène-5-yle)-glycidate d'éthyle, ces deux composés développant les caractères odorants les plus prononcés.

Parmi les composés analogues connus et ayant été précédemment décrits comme ingrédients odorants, figurent notamment l'acétyl-norbornène de formule

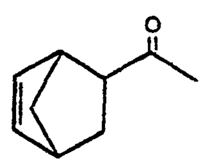

(voir brevet japonais n° 7 130, 184), ainsi que le 5-(2-alkoxy-carbonyl-vinyl)-bicyclo[2.2.1]hept-2-ène de formule

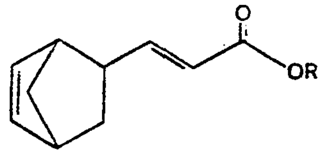

(voir DE-OS 21 63 770 publiée le 5.7.1973). Aucune indication bien définie n'a été fournie en ce qui concerne les propriétés odorantes du premier des composés précités ; quant au deuxième, il a été décrit comme un composé possédant une odeur fruitée qui rappelle celle développée par le melon.

Lorsque nous avons essayé de synthétiser le 5-(2-éthoxycarbonyl-vinyl)-bicyclo[2.2.1]hept-2-ène, suivant le procédé décrit, nous n'avons obtenu qu'un mélange contenant environ 60 % du composé en question, lequel, une fois isolé et purifié, ne présentait aucun caractère odorant « melon ».

Nous avons maintenant découvert que les composés de formule (Ia, b) possèdent par contre une note fruitée dont le caractère « melon » est fort marqué et parfaitement reproductible. De ce fait, les composés de la présente invention présentent un intérêt majeur pour l'industrie de la parfumerie, non seulement pour la manufacture de compositions parfumantes destinées à la préparation de parfums, mais également en tant qu'ingrédients actifs dans le processus de parfumage d'articles divers, tels les savons, les cosmétiques, les shampooings, les désodorisants corporels ou d'air ambiant, les poudres à lessive et

les produits d'entretien.

L'odeur des composés de l'invention est particulièrement tenace, elle est de nature fruitée avec un caractère melon dominant, voire fraise des bois ou même pêche. Le caractère olfactif global est particulièrement plaisant, frais et élégant et, par conséquent, l'utilisation de ces composés est fort étendue. Les concentrations à utiliser pour obtenir un effet olfactif tel que décrit ci-dessus peuvent varier dans une gamme très étendue, allant, par exemple, de 1 à 20, voire 30 % en poids par rapport au poids du produit parfumé considéré et selon l'effet souhaité. La valeur des concentrations est par ailleurs déterminée en fonction de la nature des coingrédients odorants dans une composition parfumante déterminée et celle des produits auxquels lesdits composés sont ajoutés, ainsi des valeurs inférieures ou supérieures à celles indiquées ci-dessus peuvent être utilisées lorsque des effets spéciaux sont désirés.

Les glycidates de l'invention s'obtiennent aisément à partir de l'acétyl-norbornène, produit commercial, conformément à un procédé qui consiste à traiter celui-ci avec un chloracétate d'alkyle en présence d'une base forte, par exemple en présence d'un alcoolate d'un métal alcalin, tel le méthylate ou le terbutylate de sodium ou de potassium. Ledit procédé est illustré par le schéma réactionnel suivant :

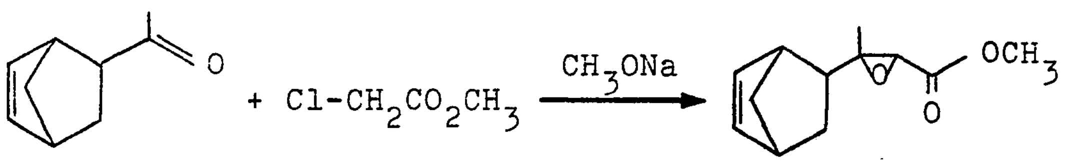


Il va sans dire que la réaction procède de façon analogue lorsqu'on remplace le chloracétate de méthyle, tel qu'indiqué dans le schéma ci-dessus, par un de ses homologues supérieurs, tel le chloracétate d'éthyle ou de butyle, pour fournir, dans ce cas, les glycidates d'éthyle ou butyle correspondants.

La formule (Ia, b) sert à désigner indiféremment les divers isomères conformationnels possibles, huit stéréoisomères peuvent en effet être théoriquement formulés. Or, le produit tel qu'obtenu par le procédé précité, est constitué par un mélange isomérique qui, comme il est indiqué plus loin pour l'ester méthylique, possède une composition bien définie et reproductible.

De tels mélanges sont parfaitement adaptés à toute utilisation pratique telle qu'envisagée dans la présente invention. Néanmoins, si cela devait s'avérer nécessaire, les différents isomères peuvent être séparés à l'aide des techniques conventionnelles, par exemple au moyen de la chromatographie gazeuse préparative.

Les composés de formule (Ib) comportant une liaison simple dans le cycle, à savoir les esters glycidiques du norbornane, peuvent être obtenus par simple réduction des dérivés correspondants insaturés, par exemple par hydrogénation catalytique en présence des catalyseurs usuels comme le palladium sur charbon actif.

La description suivante indiquera dans le détail la préparation de certains des composés définis par la formule (Ia, b)
(températures en degrés centigrades)

3-Méthyl-3-(norbornène-5-yle)-glycidate de méthyle

A une suspension de 50 g (0,367 mol) d'acétyl-norbornène et 29,7 g (0,55 mol) de méthylate de sodium, maintenue sous agitation à 0°, on a ajouté goutte à goutte 59,9 g (0,55 mol) de chloracétate de méthyle. La vitesse d'addition est telle que la température ne dépasse pas +5° et l'introduction dure ainsi 3 h environ. La température a été ensuite portée à +10° tout en maintenant l'agitation pendant 2 h, puis élevée jusqu'à température ambiante et le tout a été ensuite agité pendant une nuit. Le mélange réactionnel a été versé sur un mélange d'eau glacée (500 ml) et d'éther (220 ml) et la phase organique a été séparée, puis réunie avec les extraits organiques éthérés obtenus par extraction de la partie aqueuse (3 fractions de 50 ml d'éther). Après lavage avec une solution à 1 % d'HCl (200 ml), avec de l'eau (3 × 50 ml) et enfin avec une solution aqueuse saturée de NaCl, la phase éthérée a été séchée avec du sulfate de sodium et concentrée sous pression réduite pour fournir un résidu (70 g) qui, par distillation, a donné 53,7 g (0,258 mol) du produit désiré (rend. = 70,3 %).
IR(liquide) : 3 060, 1 752, 1 730, 1 600, 1 200, 1 080 et 1 035 $cm^{-1}$ ;
RMN($CDCl_3$) : 1,1-2,0 (8H) ; 2,6-3,1 (2H, m) ; 3,12/3,31/3,39 et 3,57 (1H, 4s) ; 3,79 (3H plusieurs s) ; 5,9-6,3 (2H, m) δ ppm
SM : $M^+$ = 208 (1) ; m/e : 190 (1), 149 (8), 131 (8), 119 (5), 105 (5), 91 (10), 83 (15), 66 (100), 55 (15), 43 (12), 41 (8), 39 (12).

En effectuant la réaction de manière analogue, mais en utilisant le chloracétate d'éthyle à la place du chloracétate de méthyle, on a obtenu l'ester éthylique correspondant, ou 3-méthyl-3-(norbornène-5-yle)-glycidate d'éthyle dont les caractères analytiques étaient les suivants :
IR(liquide) : 3 060, 1 750, 1 730, 1 605, 1 200, 1 080 et 1 035 $cm^{-1}$ ;
RMN($CDCl_3$) : 1,1-1,6 (11H, m) ; 2,6-3,1 (2H, m) ; 3,1 (2H, m) ; 3,20/3,31/3,38 et 3,56 (1H, 4s) ; 4,28 (2H) ;

5,9-6,3 (2H, m) δ ppm ;
SM : $M^+$ = 222 (2) ; m/e : 204 (2), 189 (1), 162 (5), 149 (7), 131 (6), 119 (5), 111 (6), 91 (15), 83 (17), 66 (100), 55 (15), 43 (22).

La réduction, effectuée par hydrogénation catalytique, des esters désirés du norbornène a fourni les composés saturés correspondants :

3-Méthyl-3-norbornyle-glycidate de méthyle

IR(liquide) : 1 745, 1 730, 1 305, 1 200, 1 080 et 1 025 $cm^{-1}$ ;
RMN($CDCl_3$) : 0,95-2,0 (12H) ; 2,05-2,6 (2H, 2m) ; 3,15/3,36/3,5 (1H, 3s) ; 3,79 (3H, plusieurs s) δ ppm ;
SM : $M^+$ = 210 (2) ; m/e : 192 (2), 181 (30), 151 (60), 133 (15), 122 (40), 107 (23), 93 (100), 79 (68), 67 (65), 55 (30), 43 (45) et 41 (46).

3-Méthyl-3-norbornyle-glycidate d'éthyle

IR(liquide) : 1 750, 1 730, 1 305, 1 195, 1 083 et 1 035 $cm^{-1}$ ;
RMN($CDCl_3$) : 0,9-2,0 (15H) ; 2,1-2,55 (2H, 2m) ; 3,12/3,31/3,48 (1H, 3s) et 4,25 (2H, q, J = 7,5 cps) δ ppm ;
SM : $M^+$ = 224 (4) ; m/e : 206 (3), 195 (32), 167 (22), 151 (78), 133 (20), 122 (50), 107 (25), 93 (100), 79 (60), 67 (65), 55 (30), 43 (55), 41 (46).

L'invention est illustrée de manière plus détaillée par les exemples suivants.

## Exemple 1

Poudre à lessive

On a procédé au parfumage d'une poudre à lessive du commerce, à odeur neutre, en ajoutant, à raison de 0,2 % en poids, le 3-méthyl-3-(norbornène-5-yle)-glycidate de méthyle, tel que préparé par le procédé décrit plus haut. Le produit parfumé obtenu possédait une odeur fruitée-melon fraîche et agréable.

## Exemple 2

Désodorisant d'air ambiant

15 ml d'une solution alcoolique à 4 % (éthanol 95 %) de 3-méthyl-3-(norbornène-5-yle)-glycidate de méthyle ont été introduits dans un flacon « aérosol » avec 85 ml de produit propulseur. (FRIGEN® 11/12). Le produit désodorisant ainsi obtenu permet le parfumage rapide d'une pièce.Odeur : fruitée-melon.

## Exemple 3

Une composition parfumante de base de type « Bouquet de fleurs » a été préparée en mélangeant les ingrédients suivants : (parties en poids)

| | |
|---|---|
| Alcool α-amyl-cinnamique | 150 |
| Alcool hydratropique | 100 |
| Aldéhyde α-amyl-cinnamique | 80 |
| Acétate de 3,3,5-triméthylhexyle | 80 |
| Nérol | 60 |
| Isobutyrate de citronellyle | 40 |
| Acétate de p-ter-butylcyclohexyle | 40 |
| Nonénol 1 %(*) | 40 |
| Acétate de benzyle | 30 |
| Isobutyrate de phénoxyéthyle | 20 |
| Diméthyl-phényl-éthylcarbinol | 20 |
| Géranyl-éthyl éther | 20 |
| Nonadiénol 1 %(*) | 20 |
| | 700 |

(*) dans le phtalate de diéthyle

Cette composition de base est particulièrement indiquée pour le parfumage d'un savon de toilette ou d'un shampooing, la note odorante est fraîche et fleurie.

En ajoutant à 70 g de ladite composition de base 30 g de 3-méthyl-3-(norbornène-5-yle)-glycidate de méthyle, ou d'éthyle, la note fleurie qui lui est propre se modifie en devenant une note fleurie-melon particulièrement distincte et plaisante.

Exemple 4

Le 3-méthyl-3-(norbornène-5-yle)-glycidate de méthyle a été utilisé pour parfumer des produits cosmétiques usuels tels un shampooing et une crème hydratante pour la peau à raison de 0,2 % en poids par rapport au poids total du produit parfumé terminé. Les produits ainsi obtenus possédaient une note odorante de bonne stabilité, fraîche, fruitée et particulièrement plaisante.

Le même glycidate, utilisé à raison de 5 % en poids dans l'alcool éthylique à 95 %, a servi à la préparation d'une eau-de-toilette, ainsi qu'au parfumage d'un spray d'ambiance.

En utilisant de façon analogue les esters homologues supérieurs des composés de l'invention, tel le 3-méthyl-3-(norbornène-5-yle)-glycidate de butyle, on obtient des effets similaires quoique moins prononcés.

**Revendications**

1. Composés chimiques de formule

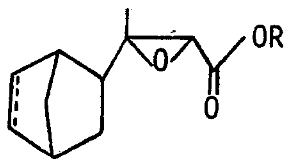

(Ia,b)

comportant une liaison simple ou double dans la position indiquée par les pointillés et dans laquelle le symbole R représente un radical alkyle inférieur comprenant 1 à 4 atomes de carbone.

2. 3-Méthyl-3-(norbornène-5-yle)-glycidate de méthyle.

3. 3-Méthyl-3-(norbornène-5-yle)-glycidate d'éthyle.

4. Utilisation des composés selon la revendication 1, en tant qu'ingrédients parfumants pour la préparation de parfums et produits parfumés.

5. Procédé pour la préparation des composés selon la revendication 1, comportant une liaison double dans la position indiquée par les pointillés, (Ia), caractérisé en ce qu'on traite l'acétyl-norbornène avec un chloracétate d'alkyle de formule

$$Cl-CH_2-CO_2R \quad (II)$$

dans laquelle R représente un radical alkyle inférieur comprenant 1 à 4 atomes de carbone, en présence d'une base forte.

6. Procédé pour la préparation des composés selon la revendication 1, comportant une liaison simple dans la position indiquée par les pointillés, (Ib), caractérisé en ce que l'on prépare les composés de formule

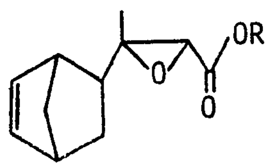

(Ia)

selon le procédé de la revendication 5 et que l'on soumet lesdits produits à une hydrogénation catalytique.

**Claims**

1. Compounds of formula

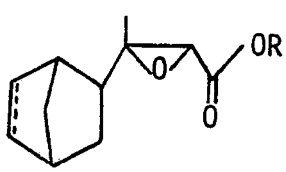

(Ia,b)

possessing a single or a double bond in the position indicated by the dotted line and wherein symbol R

represents a lower alkyl radical having 1 to 4 carbon atoms.

2. Methyl 3-methyl-3-(norbornen-5-yl)-glycidate.

3. Ethyl 3-methyl-3-(norbornen-5-yl)-glycidate.

4. Utilization of the compounds according to claim 1 as perfuming ingredients for the preparation of perfumes and perfumed products.

5. Process for the preparation of the compounds (Ia) according to claim 1, possessing a double bond in the position indicated by the dotted line, characterized in that acetyl-norbornene is reacted in the presence of a strong base, with an alkyl chloroacetate of formula

$$Cl-CH_2-CO_2R \qquad (II)$$

wherein R represents a lower alkyl radical having 1 to 4 carbon atoms.

6. Process for the preparation of the compounds according to claim 1, possessing a single bond in the position indicated by the dotted line, characterized in that the compounds of formula

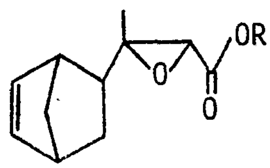

(Ia),

prepared according to the process of claim 5, are subjected to catalytic hydrogenation.

**Ansprüche**

1. Verbindungen der Formel

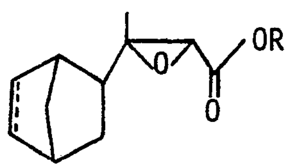

(Ia,b)

mit einer Einfach- oder einer Doppelbindung in der durch die gestrichelten Linien dargestellten Stellung, worin R eine niedrige Alkyl-Gruppe mit 1 bis 4 Kohlenstoffatomen bezeichnet.

2. Methyl 3-Methyl-3-(norbornen-5-yl)-glycidat.

3. Ethyl 3-Methyl-3-(norbornen-5-yl)-glycidat.

4. Verwendung der Verbindungen gemäss Anspruch 1, als Riechstoffe zur Herstellung von Parfümen und parfümierten Produkten.

5. Verfahren zur Herstellung der Verbindungen (Ia) gemäss Anspruch 1, mit einer Doppelbindung in der durch die gestrichelten Linien dargestellten Stellung, dadurch gekennzeichnet, dass Acetylnorbornen mit einem Alkyl Chloracetat der Formel

$$Cl-CH_2-CO_2R \qquad (II)$$

worin R eine niedrige Alkyl-Gruppe mit 1 bis 4 Kohlenstoffatomen bezeichnet, in Anwesenheit einer starken Base umsetzt.

6. Verfahren zur Herstellung der Verbindungen (Ib), gemäss Anspruch 1, mit einer Einfachbindung in der durch die gestrichelten Linien dargestellten Stellung, dadurch gekennzeichnet, dass die Verbindungen der Formel

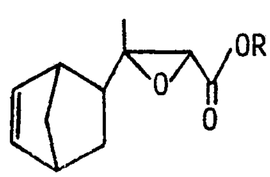

(Ia),

die gemäss dem Verfahren des Anspruchs 5 hergestellt worden sind, katalytisch hydriert.